(19) Europäisches Patentamt
European Patent Office
Office européen des brevets

(11) **EP 2 175 849 B1**

(12) # EUROPEAN PATENT SPECIFICATION

| | |
|---|---|
| (45) Date of publication and mention of the grant of the patent: **13.02.2013 Bulletin 2013/07** | (51) Int Cl.: *A61K 31/353* (2006.01)    *A61P 35/00* (2006.01) <br> *A61K 45/06* (2006.01) |
| (21) Application number: **08775866.0** | (86) International application number: **PCT/GB2008/002320** |
| (22) Date of filing: **04.07.2008** | (87) International publication number: **WO 2009/007700 (15.01.2009 Gazette 2009/03)** |

(54) **TREATMENT OF MELANOMA**

BEHANDLUNG VON MELANOM

TRAITEMENT DE MÉLANOME

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **06.07.2007 GB 0713116**
          **07.09.2007 US 967837 P**

(43) Date of publication of application:
**21.04.2010 Bulletin 2010/16**

(73) Proprietor: **e-Therapeutics plc
Long Hanborough
Oxfordshire OX29 8LN (GB)**

(72) Inventors:
• **YOUNG, Malcolm, Philip
Tyne&Wear NE2 4LZ (GB)**

• **YATES, Catherine, Mary
Tyne&Wear NE2 4LZ (GB)**
• **IDOWU, Olusola, Clement
Tyne&Wear NE2 4LZ (GB)**
• **CHARLTON, Julie, Ann
Tyne&Wear NE2 4LZ (GB)**

(74) Representative: **Gilholm, Stephen Philip
IPheions Intellectual Property
Buzzard Office
The Hawk Creative Business Park
Easingwold,
York YO61 3FE (GB)**

(56) References cited:
   **WO-A-00/24395**       **WO-A-03/077832**
   **US-A1- 2002 111 377**   **US-A1- 2006 025 419**

**Description**

**FIELD OF THE INVENTION**

The present invention provides medicaments for the treatment of melanoma. More particularly the invention provides dexanabinol, or a salt thereof, for the treatment of melanoma.

**BACKGROUND**

Incidence of melanoma cases has doubled every year since the 1940s. Melanoma is now the sixth most common cancer in men, and the seventh most common cancer in women. Its incidence is increasing in all parts of the world (Parker, S *et al,* 1997). The 5 year survival rate for melanoma is 30 to 40%, with malignant melanoma carrying the highest risk of mortality from metastasis (Jemal *et al,* 2001); Spread of the disease to distant organs such as liver, bone and brain, reduces the 5 year survival to less than 12%. There is currently no effective long-term treatment for patients with metastatic (Stage IV) melanoma. Standard chemotherapy regimens do not impart a significant long term survival benefit in these patients, and chemotherapy may be associated with a degree of morbidity due to toxicity. There is an obvious need to develop new targeted therapies for melanoma, both to prevent cancer progression and to treat advanced disease.

Finding new effective treatments for melanoma has proved very challenging. High resistance to conventional chemotherapeutic agents and radiation is a hallmark melanoma. (Smalley and Eisen, 2003; Strauss *et al.,* 2003). Research has now shifted to identifying melanoma gene mutations and the associated perturbations of signal transduction pathways, in the hope that more specific targeted therapies can be developed. Several cellular pathways important to cell proliferation, apoptosis and resistance or metastases have been shown to be activated in melanoma. Melanoma likely develops multiple defects, including loss of regulatory functions or the gain of anti-apoptotic or proliferative functions. Thus, therapeutic agents that could inhibit several signalling pathways simultaneously would be highly desirable. In addition, administration of standard chemotherapies in combination with new agents may afford the traditional chemotherapies a new lease of life in the melanoma context, if chemo resistance is inhibited. The challenge now is to develop selective agents to target these aberrant pathways.

The aberrant activation of the nuclear factor-kappa B (NF$\kappa$B) pathway has been associated with melanoma growth, metastasis and escape from apoptosis. *In vitro* studies have demonstrated constitutively elevated NF$\kappa$B binding in human melanoma cultures compared to normal melanocytes (Dhawan *et al,* 2004, McNulty *et al,* 2004). The expression of the NF$\kappa$B subunit, RelA, has also been shown to be significantly elevated in human nevi and melanomas relative to normal skin. The NF$\kappa$B pathway is therefore a potential target for treating melanoma. The constitutive activation of NF$\kappa$B has several effects. Previous studies demonstrated the persistent activation of NF$\kappa$B leads to increased chemokine production (e.g. CXCL8 and CXCL1) which stimulates angiogenesis thus promoting tumour formation in melanoma. In addition, activated NF$\kappa$B is an important central regulator of a number of genes involved in antiapoptosis and proliferation (Mayo *et al,* 1997). In addition to its anti-apoptotic role, NF$\kappa$B may also have a critical role in the development of chemoresistance in melanoma (Wang et al, 1999).

Activation of NF$\kappa$B in melanoma has been shown to be due to constitutive activation of IKK, a key regulator of the NF$\kappa$B pathway (Yang and Richmond, 2001). NF$\kappa$B pathway inhibitors, and in particular IKK inhibitors, may provide a highly effective means of killing tumour cells by targeting them towards apoptosis..

A small molecule that selectively targets NF$\kappa$B activation in melanoma without affecting NF$\kappa$B's functions in normal cells therefore has significant therapeutic potential, as a single agent or in combination with existing chemotherapies. US 2006/0025419 discloses the use of a NF$\kappa$B inhibitor (BMS-345541) for the treatment of melanoma.

One particular compound of interest is 1, 1 dimethyl heptyl-(3S, 4S)-7-hydroxy-$\Delta^6$-tetrahydrocannabinol (dexanabinol) which is disclosed in U.S. Patent No. 4,876,276.

In addition to being a non competitive NMDA receptor blocker, dexanabinol has been shown to inhibit NF$\kappa$B (Juttler et al, 2004).

**SUMMARY OF THE INVENTION**

We have now found a method of rapidly killing melanoma cells comprising contacting the cell with 1, 1 dimethyl heptyl-(3S, 4S)-7-hydroxy-. $\Delta^6$-tetrahydrocannabinol (INN dexanabinol), or a salt thereof. The present invention contemplates melanoma cancer cells that are premalignant, malignant, metastatic or multidrug-resistant

Therefore, in accordance with a first aspect, the present invention provides dexanabinol, or a salt thereof, for the treatment of melanoma.

The treatment of melanoma according to the invention may comprise inhibiting NF$\kappa$B activity in a melanoma cancer cell by providing dexanabinol, or a salt thereof, to the cell.

Alternatively, the treatment of melanoma may comprise the inhibition of tumourigenesis of a melanoma cancer cell by contacting the cell with an effective amount of dexanabinol, or a salt thereof. Inhibition of tumourigenesis includes inducing both cytotoxicity and apoptosis in the cancer cell.

Furthermore, the treatment of melanoma according to the invention may comprise separately, simultaneously or sequentially inhibiting NFκB activity and tumourigenesis of a melanoma cancer cell.

Dexanabinol, or a salt thereof, for the treatment of melanoma is advantageous, *inter alia,* because it shows reduced toxicity, reduced side effects and/or reduced resistance when compared to currently employed chemotherapeutic agents.

It is further contemplated that a second therapeutic agent may be provided in combination with dexanabinol, or a salt thereof, to a melanoma cancer cell for treatment and/or prevention of melanoma. The second agent may comprise a chemotherapeutic agent, immunotherapeutic agent, gene therapy or radio therapeutic agent. The second therapeutic agent may be administered with the dexanabinol, or a salt thereof, separately, simultaneously or sequentially.

Suitable salts of dexanabinol are well known and are described in the prior art. Salts of organic and inorganic acids and bases that may be used to make pharmaceutically acceptable salts. Such acids include, without limitation, hydrofluoric, hydrochloric, hydrobromic, hydroiodic, sulphuric, nitric, phosphoric, citric, succinic, maleic, and palmitic acids. The bases include such compounds as sodium and ammonium hydroxides. Those skilled in the art are familiar with quaternizing agents that can be used to make pharmaceutically acceptable quaternary ammonium derivatives of dexanabinol. These include without limitation methyl and ethyl iodides and sulphates.

Accordingly, and in one embodiment, the present invention provides a use of dexanabinol, or a salt thereof, in the manufacture of a medicament for the treatment of melanoma.

We especially provide a use of dexanabinol, or a salt thereof, in the manufacture of a topically administrable medicament, e.g. a topically administrable medicament for the treatment of melanoma.

Furthermore, in a second aspect, the present invention provides a compound for use in a method of treatment melanoma, said method comprising the administration of a therapeutically effective amount of dexanabinol and salts and/or combinations thereof.

Dexanabinol and salts and/or combinations thereof are known *per se* and may be prepared using methods known to the person skilled in the art or may be obtained commercially. In particular, dexanabinol and methods for its preparation are disclosed in U.S. Patent No. 4,876,276.

Advantageously, the compound and salts and/or combination thereof may be administered orally, or intravenously.

Thus, the compound may be put up as a tablet, capsule, dragee, suppository, suspension, solution, injection, e.g. intravenously, intramuscularly or intraperitoneally, implant, a topical, e.g. transdermal, preparation such as a gel, cream, ointment, aerosol or a polymer system, or an inhalation form, e.g. an aerosol or a powder formulation.

Compositions suitable for oral administration include tablets, capsules, dragees, liquid suspensions, solutions and syrups;

Compositions suitable for topical administration to the skin include creams, e.g. oil-in-water emulsions, water-in-oil emulsions, ointments, gels, lotions, unguents, emollients, colloidal dispersions, suspensions, emulsions, oils, sprays, foams, mousses, and the like. Compositions suitable for topical application may also include, for example, liposomal carriers made up of lipids or special detergents.

Examples of other adjuvants, diluents or carriers are:

for tablets and dragees - fillers, e.g. lactose, starch, microcrystalline cellulose, talc and stearic acid; lubricants/glidants, e.g. magnesium stearate and colloidal silicon dioxide; disintegrants, e.g. sodium starch glycolate and sodium carboxymethylcellulose;

for capsules - pregelatinised starch or lactose;

for oral or injectable solutions or enemas - water, glycols, alcohols, glycerine, vegetable oils;

for suppositories - natural or hardened oils or waxes.

It may be possible to administer the compound or salts and/or combination thereof or any combined regime as described above, transdermally via, for example, a transdermal delivery device or a suitable vehicle or, e.g. in an ointment base, which may be incorporated into a patch for controlled delivery. Such devices are advantageous, as they may allow a prolonged period of treatment relative to, for example, an oral or intravenous medicament.

Examples of transdermal delivery devices may include, for example, a patch, dressing, bandage or plaster adapted to release a compound or substance through the skin of a patient. A person of skill in the art would be familiar with the materials and techniques which may be used to transdermally deliver a compound or substance and exemplary transdermal delivery devices are provided by GB2185187, US3249109, US3598122, US4144317, US4262003 and US4307717.

For the treatment of melanoma, the invention especially allows administration of compositions topically on the skin or by injection, e.g. subcutaneously, or both. In additional the dexanabinol, or a salt thereof, may be delivered simultaneously or sequentially by an injection method and topically to reduce the growth of a skin tumour.

Topically administered compositions are especially preferred.

The invention will now be illustrated by way of example only.

## DETAILED DESCRIPTION

### Example 1

### Induction of Apoptosis by Dexanabinol in Human Melanoma Cell Lines

### Methods

3 human melanoma cell lines (A375, G-361, WM266-4) were maintained in RPMI 1640 culture medium (Sigma, UK) containing 10% (v/v) heat inactivated foetal bovine serum (Sigma, UK) and 2 mM L-glutamate at 37°C in 5% humidified $CO_2$.

Cells were harvested, washed, re-suspended into growth medium and counted (Beckman-Coulter Vi-CELL XR). Cells were plated onto the middle 240 wells of 384 tissue culture plates at $1.6 \times 10^5$ to $2.4 \times 10^5$ cells/ml in $12.5\mu$l/ well aliquots. $50\mu$l of growth media was aliquoted into the outer wells. 2 plates were prepared per cell line. Plates were incubated overnight at 37°C, in 5% humidified $CO_2$.

Dexanabinol was prepared in growth medium at 2 times the final assay concentration at 125, 31.3, 7.81, 2.00, 0.49, 0.12, 0.031 and $0.008\mu$M (DMSO concentration was kept constant across the dilution range at 0.5%).

Cisplatin was used as a positive control. The final assays concentrations were 10, 2.5, 0.63, 0.156, 0.039, 0.010, 0.002 and $0.0006\mu$g/ml. $12.5\mu$l per well of dexanabinol or cisplatin dilutions were added to the plates in replicates of 6. $12.5\mu$l of growth media was added to the media control wells. The plates were incubated for 24 hours at 37°C, in 5% humidified $CO_2$.

Caspase 3/7 levels were assessed by Apo-ONE© Homogeneous Caspase- 3/7 assay kit. Flourescence was measured using a FlexStation© II[384] plate reader at 1, 2, 3 and 4 hours after addition of the caspase substrate. The 4 hour readings were used for analysis.

The cell viability assay was performed in parallel on the same plate for each line using CellTiter-Blue© (Promega) reagent. Briefly, $25\mu$l of CellTiter-Blue© (Promega) reagent was added to each well. The plates were shaken for 1 minute at 500 rpm and then incubated at 37°C, 5% $CO_2$ for 4 hours. Fluorescence was measured using a FlexStation© II[384] plate reader (570nm excitation wavelength, 600nm emission wavelength, 590nm cut-off.) The plots showing the cytotoxic effect of dexanabinol and cisplatin are shown as an overlay on the same graph.

Results

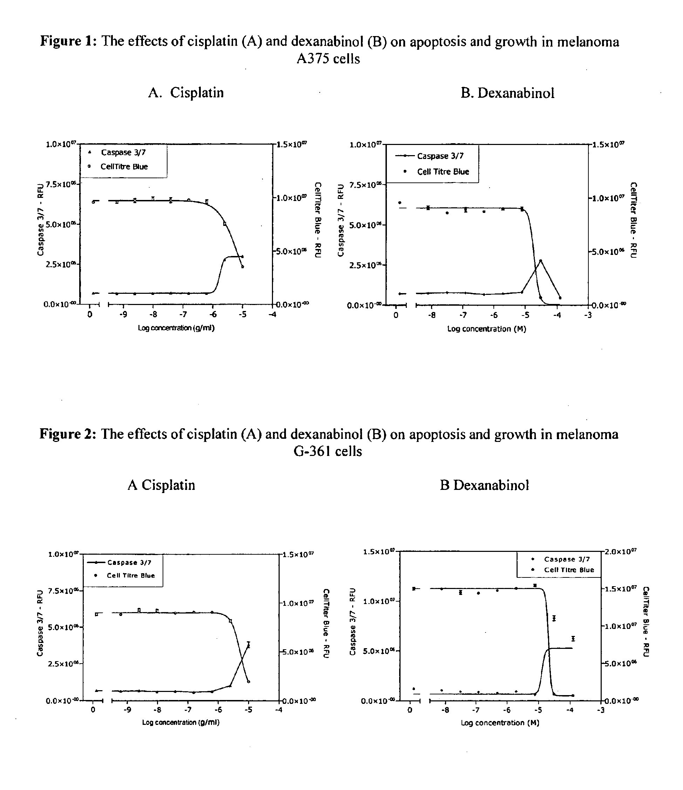

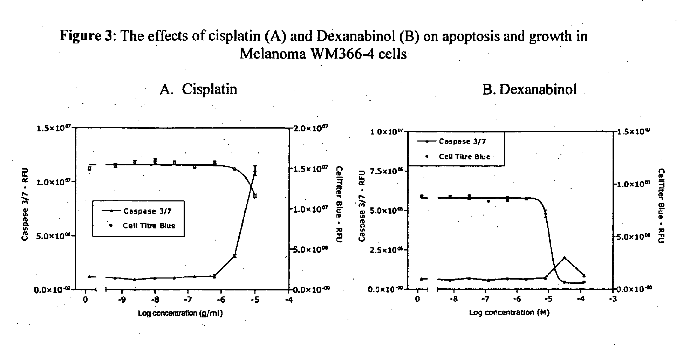

The induction of apoptosis in A375, G-361 and WM266-4 melanoma cell lines following 24 hours incubation with either cisplatin or dexanabinol is shown in Figures 1-3 respectively and summarized in Table 1. The assessment of cell viability as measured by the CellTiter-Blue® assay, indicating cytotoxicity is also shown.

Cisplatin was used as a positive control and a cytotoxic response was observed in all 3 melanoma cell lines with an approximate $IC_{50}$ value of 20-60$\mu$M. The induction of apoptosis was not as easily quantified due to inadequate dose curves (G-361, WM266-4).

Dexanabinol induced a cytotoxic response with $IC_{50}$ values in the range of 10-21$\mu$M in the 3 melanoma cell lines. The induction of apoptosis was not quantified in G-631 and A375 due to inadequate dose response curves. A peak response in apoptosis occurred at 2.5$\mu$M and dropped at the highest concentration of 10$\mu$M possibly due to cell lysis and loss.

**Table 1:** Effect of Dexanabinol on induction of apoptosis and cell proliferation in 3 human melanoma cell lines.

| Cell line | Cisplatin | | Dexanabinol | |
|---|---|---|---|---|
| | ↑Apoptosis EC$_{50}$ ($\mu$M) | ↓Viability IC$_{50}$ ($\mu$M) | ↑Apoptosis EC$_{50}$ ($\mu$M) | ↓Viability IC$_{50}$ ($\mu$M) |
| A375 | 5.67** | 21.8** | ND*** | 19.16** |
| G-361 | Approx 33.3-100** | 18.00** | ND* | 10.97*** |

(continued)

| Cell line | Cisplatin | | Dexanabinol | |
| --- | --- | --- | --- | --- |
| | ↑Apoptosis EC$_{50}$ ($\mu$M) | ↓Viability IC$_{50}$ ($\mu$M) | ↑Apoptosis EC$_{50}$ ($\mu$M) | ↓Viability IC$_{50}$ ($\mu$M) |
| WM266-4 | Approx 33.3-100*** | 62.00* | 13.04*** | 20.87** |
| ND - EC/IC$_{50}$ not determined due to inadequate dose response curve<br>Rank * weak apoptosis induction and decrease in proliferation (<35%)<br>** moderate apoptosis induction and decrease in proliferation (35-70%)<br>*** good apoptosis induction and decrease in proliferation (>70%) | | | | |

The results are illustrated in Figures 1 to 3, in which;

Figure 1 illustrates the effects of cisplatin (A) and dexanabinol (B) on apoptosis and growth in melanoma A375 cells;
Figure 2 illustrates the effects of cisplatin (A) and dexanabinol (B) on apoptosis and growth in melanoma G-361 cells; and
Figure 3 illustrates the effects of cisplatin (A) and Dexanabinol (B) on apoptosis and growth in Melanoma WM366-4 cells.

## Example 2

### Inhibition of Melanoma Cell Proliferation by Dexanabinol

### Methods

The ability of dexanabinol to inhibit melanoma cell growth was also studied in 3 melanoma cell lines (A375, UACC62, Malme-3M) using the Sulforhodamine B (SRB) assay.
Sterile solutions of the final concentrations of dexanabinol were prepared (0.001$\mu$M to 100$\mu$M in 0.5% DMSO).
The cells were incubated with drug concentrations in 0.5% DMSO (100ul total volume per well) at 37 °C in 5 % $CO_2$ for 5 days. Control wells contained cells plus 0.5% DMSO plus medium.
The SRB growth inhibition assay was conducted over 24 and 5 day exposure periods. Following exposure, the cells were fixed, stained with SRB and read on a SpectorMax® 250 microplate spectrophotometer system plate reader.

### Results

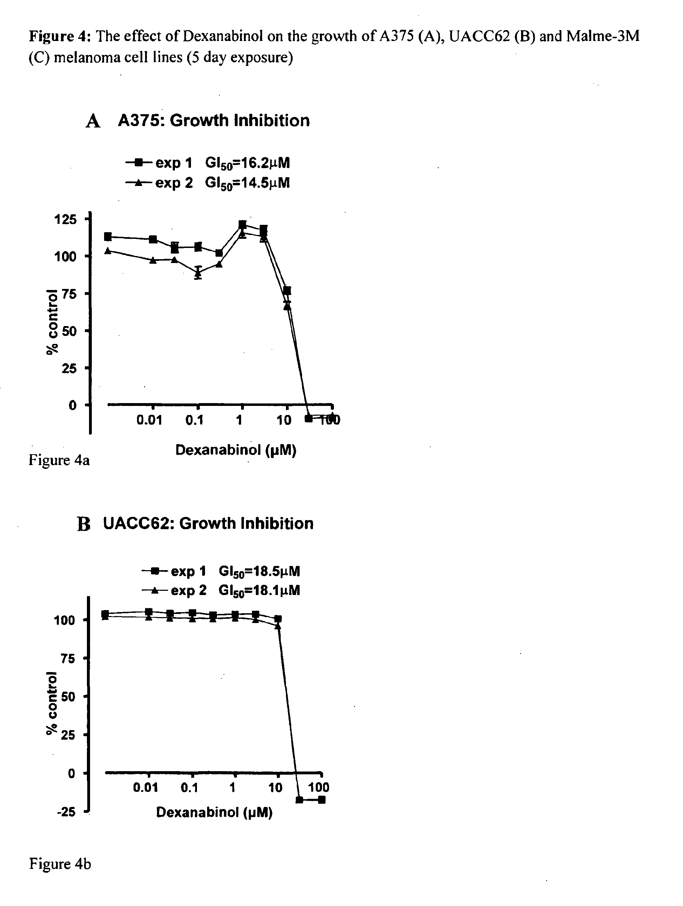

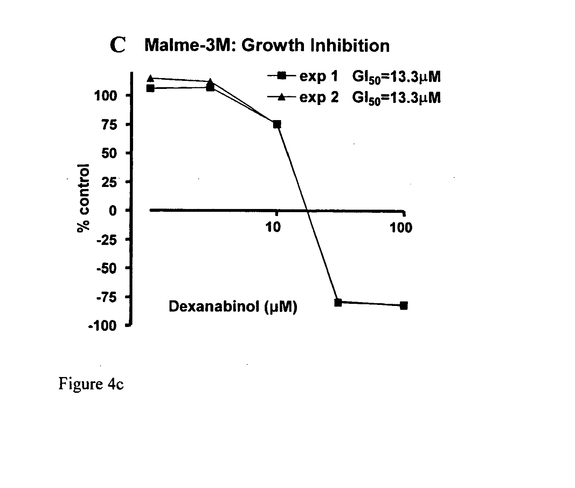

The effect of dexanabinol on the inhibition of growth in 3 melanoma cell lines after 5 days exposure is shown in Figure 4. Growth inhibition was also measured after 24 hours exposure. GI$_{50}$ values for both exposure times are summarised in Table 2.

Table 2: Effect of exposure time on dexanabinol's ability to inhibit melanoma cell growth in vitro.

| | | A375 | Malme-3M | UACC62 |
| --- | --- | --- | --- | --- |
| GI$_{50}$($\mu$M)[a] | 5-day | 16.2<br>14.4 | 13.3<br>13.3 | 18.5<br>18.1 |
| | 24-hour | 14.3 | | |
| [a] Measured by SRB growth inhibition assay | | | | |

## Example 3

### Time-Course Effect of Dexanabinol on Cell Proliferation

### Methods

The time-course effect of dexanabinol on cell proliferation in one melanoma cell line, A375, was examined using the clonogenic assay method.
A375 cells were harvested from the growing cell culture and counted. Cells were diluted to 1.0x10$^5$/ml and

seeded 2 ml/well to 6 well plates.

The cells were incubated overnight at 37°C in humidified incubator 95% air + 5% $CO_2$.

The cells were treated with dexanabinol at 4 doses up to 20$\mu$M at 3 exposure periods (1 hour, 6 hours and 24 hours).

After each exposure period, the cells were harvested from the wells by washing twice with PBS then adding 0.2ml of single strength trypsin to each well and incubating at 37°C until the cells detached. Cell suspensions were counted at 1/10 dilution and then diluted to 1/10, 1/100, 1/1000. 10 cm dishes (containing 7 ml fresh medium) were seeded with 3 different cell densities for dexanabinol treatment and control treatment. When colonies of suitable size formed in the control dishes, dishes were fixed, stained and counted.

Percentage survival in the dexanabinol treated A375 cells was calculated using the following equation:

$$\text{Cloning efficiency \%} = \frac{\text{Colonies counted}}{\text{Cells seeded}} \times 100$$

$$\text{\% Survival} = \frac{\text{Drug treated cell cloning efficiency}}{\text{Control cell cloning efficiency}} \times 100$$

**Results**

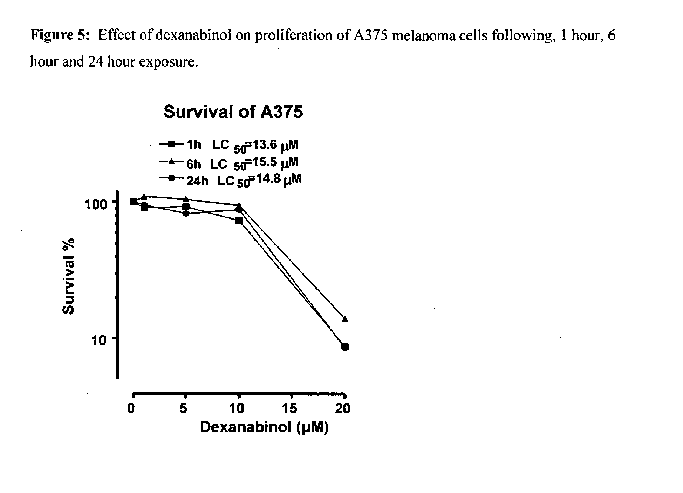

The time-course effect of dexanabinol on cell killing in A375 melanoma cells is shown in Figure 5.

**Example 4**

**Inhibition of Human Melanoma Cell Xenograft Growth by Dexanabinol**

**Methods:**

Having established that dexanabinol inhibited melanoma cell proliferation *in vitro,* we then sought to establish whether the compound was active *in vivo.* A preliminary pharmacokinetic (PK) and maximum tolerated dose (MTD) study was carried out to determine whether therapeutically effective dose levels of dexanabinol could be achieved in CD1 A375 tumour-bearing mice. The results demonstrated that at the MTD (100 mg/kg i.v. single dose) a plasma concentration of 10$\mu$M was achievable for 2 hours. On the basis of this, a single-dose efficacy study was undertaken.

Dexanabinol was diluted in a vehicle of 10 % Cremophor/Ethanol (1:1 v/v) in saline. Control mice received vehicle alone.

10 mice received 100 mg/kg i.v. dexanabinol. 10 mice received Vehicle (10 ml/kg) Mice were implanted with 1 x $10^7$ A375 human melanoma cells, in 50 $\mu$l of medium, on the flank. Once tumours were palpable (approx 5 mm x 5 mm) 20 mice were treated with either dexanabinol or vehicle (as outlined above). Mice were observed at least daily and weighed 3 times a week. Tumour size was measured 3 times a week after treatment. The treatment lasted 4 weeks

**Results**

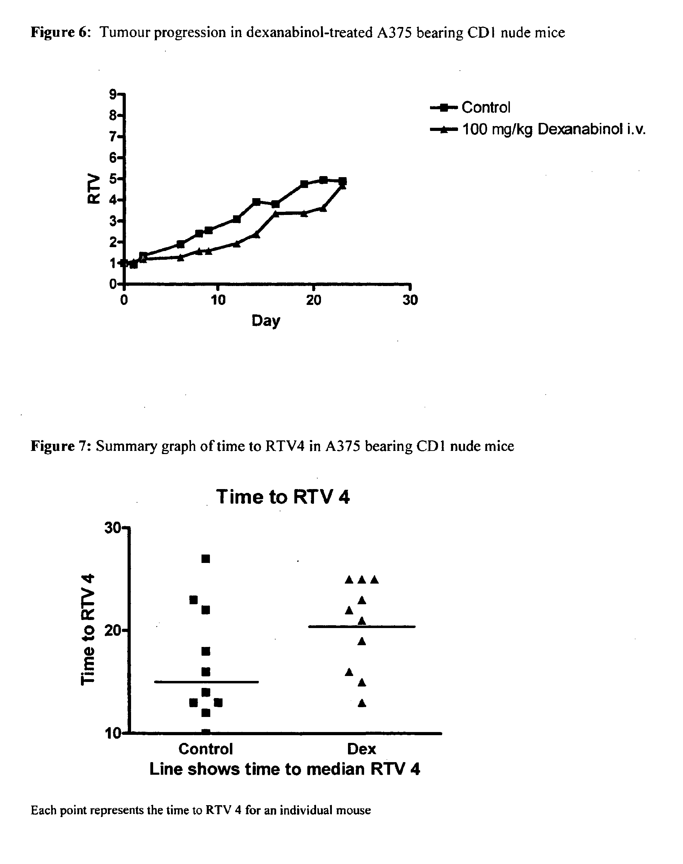

The time course of tumour growth and a summary graph of the time for tumours to reach a tumour volume 4 times that on the day of treatment (time to RTV 4) are shown in Figures 6 and 7.

**SUMMARY**

Dexanabinol was tested against several established human melanoma cell lines, including some derived from disseminated melanoma metastases in other tissues. The *in vitro* cell proliferation assays showed dexanabinol to be profoundly cytotoxic to all tested human melanoma cell lines at concentrations distributed about a mean of 14 $\mu$M. Dexanabinol induced apoptotic cell death in a caspase 3/7 dependent manner.

This effect was time-independent, with cell killing being as profound after 1 hour as after 24 hours.

The *in vitro* anti-tumour effects of dexanabinol were observed at drug concentrations demonstrated to be safe and clinically achievable in patients (~10-20$\mu$M). The effect of a single dose of dexanabinol on human melanoma cell xenograft growth was then undertaken. The minimum required plasma concentration, based on the *in vitro* cell proliferation

assay results, (10μM maintained for at least 2 hours) was achieved by administering a single i.v. dose of dexanabinol at the MTD (100 mg/kg) in CD1 nude mice. The single dose of dexanabinol had a growth delaying effect in CD1 nude mice bearing the A375 human tumour xenograft.

**Claims**

1. Dexanabinol, or a salt thereof, for use in the treatment of melanoma.

2. Dexanabinol for use according to claim 1 wherein the melanoma cancer cells are premalignant, malignant, metastatic or multidrug-resistant.

3. Dexanabinol for use according to claim 1 wherein the treatment of melanoma comprises separately, simultaneously or sequentially inhibiting NFκB activity in a melanoma cancer cell.

4. Dexanabinol for use according to claim 1 wherein the treatment of melanoma comprises inhibition of tumourigenesis of a melanoma cancer cell.

5. Dexanabinol for use according to claim 4 wherein the inhibition of tumourigenesis includes inducing both cytotoxicity and apoptosis in the cancer cell.

6. Dexanabinol for use according to claim 1 in combination with a second therapeutic agent selected from one or more of a chemotherapeutic agent, an immunotherapeutic agent, a gene therapy or a radiotherapeutic agent.

7. The use of dexanabinol in the manufacture of a medicament for the treatment or alleviation of melanoma.

8. The use according to claim 7 wherein the melanoma cancer cells are premalignant, malignant, metastatic or multidrug-resistant.

9. The use according to claim 7 wherein the treatment of melanoma comprises separately, simultaneously or sequentially inhibiting NFκB activity in a melanoma cancer cell.

10. The use according to claim 7 wherein the treatment of melanoma comprises inhibition of tumourigenesis of a melanoma cancer cell.

11. The use according to claim 10 wherein the inhibition of tumourigenesis includes inducing both cytotoxicity and apoptosis in the cancer cell.

12. The use according to claim 7 which in the manufacture of a combination therapy comprising dexanabinol with one or more of a therapeutic agent comprising a chemotherapeutic agent, an immunotherapeutic agent, a gene therapy, or a radiotherapeutic agent; separately, simultaneously or sequentially.

13. The use according to claim 7 wherein the medicament is for oral or intravenous administration.

14. A pharmaceutical composition comprising dexanabinol, or a salt thereof, and one or more of a therapeutic agent comprising a chemotherapeutic agent, an immunotherapeutic agent, a gene therapy, or a radiotherapeutic agent; in admixture with a pharmaceutically acceptable adjuvant, diluent or carrier for use in the treatment or alleviation of melanoma.

15. A pharmaceutical composition according to claim 14 for use according to claim 14 adapted for topical or intravenous administration.

**Patentansprüche**

1. Dexanabinol, oder dessen Salz, zur Verwendung bei der Behandlung eines Melanoms.

2. Dexanabinol zur Verwendung gemäß Anspruch 1, wobei die Melanomkrebszellen präkanzerös, kanzerös, metastatisch oder mehrfach medikamentenresistent sind.

3. Dexanabinol zur Verwendung gemäß Anspruch 1, wobei die Behandlung eines Melanoms eine getrennt, gleichzeitig oder sequentiell hemmende NFκB-Aktivität in einer Melanomkrebszelle umfasst.

4. Dexanabinol zur Verwendung gemäß Anspruch 1, wobei die Behandlung eines Melanoms die Hemmung der Tumorgenese von Melanomkrebszellen umfasst.

5. Dexanabinol zur Verwendung gemäß Anspruch 4, wobei die Hemmung von Tumorgenese das Induzieren sowohl von Zytotoxizität als auch Apoptosis in der Krebszelle umfasst.

6. Dexanabinol zur Verwendung gemäß Anspruch 1 in Verbindung mit einem zweiten therapeutischen Wirkstoff, der aus einem oder mehreren eines chemotherapeutischen Wirkstoffs, eines immuntherapeutischen Wirkstoffs, einer Gentherapie oder eines radiotherapeutischen Wirkstoffs ausgewählt ist.

7. Verwendung von Dexanabinol bei der Herstellung eines Medikamentes zur Behandlung oder Linderung eines Melanoms.

8. Verwendung gemäß Anspruch 7, wobei die Melanomkrebszellen präkanzerös, kanzerös, metastatisch oder mehrfach medikamentenresistent sind.

9. Verwendung gemäß Anspruch 7, wobei die Behandlung eines Melanoms eine getrennt, gleichzeitig oder sequentiell hemmende NFκB-Aktivität in einer Melanomkrebszelle umfasst.

10. Verwendung gemäß Anspruch 7, wobei die Behandlung eines Melanoms die Hemmung der Tumorgenese einer Melanomkrebszelle umfasst.

11. Verwendung gemäß Anspruch 10, wobei die Hemmung der Tumorgenese das Induzieren sowohl von Zytotoxizität als auch Apoptosis in der Krebszelle umfasst.

12. Verwendung gemäß Anspruch 7, die bei der Herstellung einer Kombinationstherapie Dexanabinol mit einem oder mehreren eines therapeutischen Wirkstoffs umfasst, der einen chemotherapeutischen Wirkstoff, einen immuntherapeutischen Wirkstoff, eine Gentherapie oder einen radiotherapeutischen Wirkstoff getrennt, gleichzeitig oder sequentiell enthält.

13. Verwendung gemäß Anspruch 7, wobei das Medikament zur oralen oder intravenösen Verabreichung vorgesehen ist.

14. Pharmazeutische Zusammensetzung mit Dexanabinol, oder dessen Salz, und einem oder mehreren eines therapeutischen Wirkstoffs, der einen chemotherapeutischen Wirkstoff, einen immuntherapeutischen Wirkstoff, eine Gentherapie oder einen radiotherapeutischen Wirkstoff umfasst, in einer Mischung mit einem pharmazeutisch zulässigen Hilfsmittel, Verdünnungsmittel oder Trägerstoff zur Verwendung bei der Behandlung oder Linderung eines Melanoms.

15. Pharmazeutische Zusammensetzung gemäß Anspruch 14 zur Verwendung gemäß Anspruch 14, die zur lokalen oder intravenösen Verabreichung geeignet ist.

**Revendications**

1. Dexanabinol, ou un sel de celui-ci, pour l'utilisation en vue du traitement du mélanome.

2. Dexanabinol pour l'utilisation selon la revendication 1, **caractérisé en ce que** les cellules cancereuses de mélanome sont pré-malignes, malignes, à l'état de métastase ou résistantes à plusieurs médicaments.

3. Dexanabinol pour l'utilisation selon la revendication 1, **caractérisé en ce que** le traitement du mélanome comporte séparément, simultanément ou séquentiellement le fait d'inhiber l'activité NFκB dans une cellule cancéreuse de mélanome.

**4.** Dexanabinol pour l'utilisation selon la revendication 1, **caractérisé en ce que** le traitement du mélanome comporte l'inhibition de la genèse tumorale d'une cellule cancéreuse de mélanome.

**5.** Dexanabinol pour l'utilisation selon la revendication 4, **caractérisé en ce que** l'inhibition de la genèse tumorale inclut le fait d'induire à la fois la cytotoxicité et l'apoptose dans la cellule de cancer.

**6.** Dexanabinol pour l'utilisation selon la revendication 1, en combinaison avec un second agent thérapeutique choisi parmi un ou plusieurs d'un agent chimiothérapeutique, un agent immunothérapeutique, un agent de thérapie de gènes ou un agent radiothérapeutique.

**7.** Utilisation de dexanabinol dans la fabrication d'un médicament pour le traitement ou l'atténuation du mélanome.

**8.** Utilisation selon la revendication 7, **caractérisée en ce que** les cellules de cancer mélanome sont pré-malignes, malignes, à l'état de métastase ou résistantes à plusieurs médicaments.

**9.** Utilisation selon la revendication 7, **caractérisée en ce que** le traitement du mélanome comporte séparément, simultanément ou séquentiellement, le fait d'inhiber l'activité NFκB dans une cellule cancéreuse de mélanome.

**10.** Utilisation selon la revendication 7, **caractérisée en ce que** le traitement de mélanome comporte l'inhibition de la genèse tumorale d'une cellule cancéreuse de mélanome.

**11.** Utilisation selon la revendication 10, **caractérisée en ce que** l'inhibition de la genèse tumorale inclut le fait d'induire à la fois la cytotoxicité et l'apoptose dans la cellule de cancer.

**12.** Utilisation selon la revendication 7, dans une thérapie comportant du dexanabinol en combinaison avec un ou plusieurs des agents parmi un agent chiomiothérapeutique, un agent immuno thérapeutique, un agent de thérapie de gènes, ou un agent radio thérapeutique, séparément, simultanément ou séquentiellement.

**13.** Utilisation selon la revendication 7, **caractérisée en ce que** le médicament est administré par voie ou intraveineuse.

**14.** Composition pharmaceutique comprenant du dexanabinol, ou un sel de celui-ci, et un ou plusieurs agents thérapeutiques parmi un agent chiomiothérapeutique, un agent immunothérapeutique, un agent de thérapie de gènes, ou un agent radiothérapeutique, en adjonction avec un adjuvant, diluant ou porteur, acceptable sur le point pharmaceutique, pour l'utilisation dans le traitement ou l'atténuation du mélanome.

**15.** Composition pharmaceutique selon la revendication 14, pour l'utilisation selon la revendication 14, adaptée pour une administration topique ou intraveineuse.

Figure 1: The effects of cisplatin (A) and dexanabinol (B) on apoptosis and growth in melanoma
A375 cells

A. Cisplatin

B. Dexanabinol

Figure 2: The effects of cisplatin (A) and dexanabinol (B) on apoptosis and growth in melanoma
G-361 cells

A Cisplatin

B Dexanabinol

Figure 3: The effects of cisplatin (A) and Dexanabinol (B) on apoptosis and growth in
Melanoma WM366-4 cells

A.  Cisplatin

B. Dexanabinol

**Figure 4:** The effect of Dexanabinol on the growth of A375 (A), UACC62 (B) and Malme-3M (C) melanoma cell lines (5 day exposure)

## A A375: Growth Inhibition

■ exp 1  $GI_{50}$=16.2μM
▲ exp 2  $GI_{50}$=14.5μM

Dexanabinol (μM)

Figure 4a

## B UACC62: Growth Inhibition

■ exp 1  $GI_{50}$=18.5μM
▲ exp 2  $GI_{50}$=18.1μM

Dexanabinol (μM)

Figure 4b

C   Malme-3M: Growth Inhibition

Figure 4c

**Figure 5:** Effect of dexanabinol on proliferation of A375 melanoma cells following, 1 hour, 6 hour and 24 hour exposure.

## Survival of A375

—■— 1h  LC$_{50}$=13.6 μM
—▲— 6h  LC$_{50}$=15.5 μM
—●— 24h  LC$_{50}$=14.8 μM

**Figure 6:** Tumour progression in dexanabinol-treated A375 bearing CD1 nude mice

**Figure 7:** Summary graph of time to RTV4 in A375 bearing CD1 nude mice

Each point represents the time to RTV 4 for an individual mouse

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060025419 A
- US 4876276 A
- GB 2185187 A
- US 3249109 A
- US 3598122 A
- US 4144317 A
- US 4262003 A
- US 4307717 A